(19) Europäisches Patentamt / European Patent Office / Office européen des brevets

(11) **EP 3 319 989 B1**

(12) **FASCICULE DE BREVET EUROPEEN**

(45) Date de publication et mention de la délivrance du brevet:
**08.01.2025 Bulletin 2025/02**

(21) Numéro de dépôt: **16747826.2**

(22) Date de dépôt: **06.07.2016**

(51) Classification Internationale des Brevets (IPC):
**C07K 16/00** (2006.01)

(52) Classification Coopérative des Brevets (CPC):
**A61P 37/06; C07K 16/00;** C07K 2317/52;
C07K 2317/70; C07K 2317/94

(86) Numéro de dépôt international:
**PCT/FR2016/051708**

(87) Numéro de publication internationale:
**WO 2017/006052 (12.01.2017 Gazette 2017/02)**

(54) **UTILISATION DE FRAGMENTS FC MODIFIES EN IMMUNOTHERAPIE**

VERWENDUNG VON MODIFIZIERTEN FRAGMENTEN IN DER IMMUNTHERAPIE

USE OF MODIFIED FC FRAGMENTS IN IMMUNOTHERAPY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.07.2015 FR 1556399**

(43) Date de publication de la demande:
**16.05.2018 Bulletin 2018/20**

(73) Titulaire: **Laboratoire Français du Fractionnement et des Biotechnologies**
**92800 Puteaux (FR)**

(72) Inventeurs:
• **MONDON, Philippe**
  **62840 Neuve Chapelle (FR)**
• **MONNET-MARS, Céline**
  **59130 Lambersart (FR)**
• **FONTAYNE, Alexandre**
  **59110 La Madeleine (FR)**
• **DE ROMEUF, Christophe**
  **59130 Lambersart (FR)**
• **CHTOUROU, Abdessatar**
  **78990 Elancourt (FR)**

(74) Mandataire: **Lavoix**
**2, place d'Estienne d'Orves**
**75441 Paris Cedex 09 (FR)**

(56) Documents cités:
EP-A1- 2 233 500          EP-A1- 2 233 500
EP-A1- 2 233 500          WO-A1-2013/095966
WO-A1-2013/163630         WO-A1-2015/100299
WO-A2-2008/151088         US-A1- 2008 206 246

• **CELINE MONNET ET AL: "Selection of IgG variants with increased FcRn binding using random and directed mutagenesis: impact on effector functions", FRONTIERS IN IMMUNOLOGY, vol. 6, no. 39, 4 February 2015 (2015-02-04), pages 1 - 14, XP055238838, DOI: 10.3389/fimmu.2015.00039**
• **MONNET C ET AL: "Combined glyco- and protein-Fc engineering simultaneously enhance cytotoxicity and half-life of a therapeutic antibody", MABS, LANDES BIOSCIENCE, US, vol. 6, no. 2, 1 March 2014 (2014-03-01), pages 422 - 436, XP002738427, ISSN: 1942-0862, [retrieved on 20140115], DOI: 10.4161/ MABS.27854**

- ROBERT L SHIELDS ET AL: "High resolution mapping of the binding site on human IgG1 for Fc gamma RI, Fc gamma RII, Fc gamma RIII and FcRn and design of IgG1 variants with improved binding to the Fc gamma R", JOURNAL OF BIOLOGICAL CHEMISTRY, AMERICAN SOCIETY FOR BIOCHEMISTRY AND MOLECULAR BIOLOGY, US, vol. 276, no. 9, 2 March 2001 (2001-03-02), pages 6591 - 6604, XP002638208, ISSN: 0021-9258, [retrieved on 20001128], DOI: 10.1074/JBC.M009483200
- A. M. GOETZE ET AL: "High-mannose glycans on the Fc region of therapeutic IgG antibodies increase serum clearance in humans", GLYCOBIOLOGY, vol. 21, no. 7, 1 July 2011 (2011-07-01), pages 949 - 959, XP055169019, ISSN: 0959-6658, DOI: 10.1093/glycob/cwr027
- TIMO RATH ET AL: "Fc-fusion proteins and FcRn: structural insights for longer-lasting and more effective therapeutics", CRITICAL REVIEWS IN BIOTECHNOLOGY, vol. 35, no. 2, 24 October 2013 (2013-10-24), US, pages 235 - 254, XP055597699, ISSN: 0738-8551, DOI: 10.3109/ 07388551.2013.834293
- MATHILDE BAS ET AL: "Fc Sialylation Prolongs Serum Half-Life of Therapeutic Antibodies", THE JOURNAL OF IMMUNOLOGY, vol. 202, no. 5, 1 March 2019 (2019-03-01), US, pages 1582 - 1594, XP055758310, ISSN: 0022-1767, DOI: 10.4049/ jimmunol.1800896
- PHILIPPE MONDON: "An innovative monomeric Fc fragment with high binding to FcRn and Fc gamma receptors for the treatment of autoimmune diseases", 1 June 2019 (2019-06-01), XP055758563, Retrieved from the Internet <URL:https://epo.summon. serialssolutions. com/2.0.0/link/0/eLvHCXMwrV07TwMxDI5ALDCB QOlpeWDkyl2SNpdKCKGKAhlTgoHplldz6tBrVQ ob_x376BU2Fi7LDZYVOUry2bE_C3EesaATQKU sBRUyLYPMHKGMLO_nwealK3xbFPY60s935ul OP26luquFwTbzDHvcO8rRpu9hxyd0Of-JY12vzPoQrziRMhA6q-gbDpPxyiFda2gHLlqmZi mLgEnaqPoey03aApzR07lPKXluLhd1T_KwWuu eMqb> [retrieved on 20201209], DOI: 10.13140/ rg.2.2.29444.37760
- CELINE MONNET ET AL: "Selection of IgG variants with increased FcRn binding using random and directed mutagenesis: impact on effector functions", FRONTIERS IN IMMUNOLOGY, vol. 6, no. 39, 4 February 2015 (2015-02-04), pages 1 - 14, XP055238838, DOI: 10.3389/fimmu.2015.00039
- MONNET C ET AL: "Combined glyco- and protein-Fc engineering simultaneously enhance cytotoxicity and half-life of a therapeutic antibody", MABS, LANDES BIOSCIENCE, US, vol. 6, no. 2, 1 March 2014 (2014-03-01), pages 422 - 436, XP002738427, ISSN: 1942-0862, [retrieved on 20140115], DOI: 10.4161/ MABS.27854
- ROBERT L SHIELDS ET AL: "High resolution mapping of the binding site on human IgG1 for Fc gamma RI, Fc gamma RII, Fc gamma RIII and FcRn and design of IgG1 variants with improved binding to the Fc gamma R", JOURNAL OF BIOLOGICAL CHEMISTRY, AMERICAN SOCIETY FOR BIOCHEMISTRY AND MOLECULAR BIOLOGY, US, vol. 276, no. 9, 2 March 2001 (2001-03-02), pages 6591 - 6604, XP002638208, ISSN: 0021-9258, [retrieved on 20001128], DOI: 10.1074/JBC.M009483200
- A. M. GOETZE ET AL: "High-mannose glycans on the Fc region of therapeutic IgG antibodies increase serum clearance in humans", GLYCOBIOLOGY, vol. 21, no. 7, 1 July 2011 (2011-07-01), pages 949 - 959, XP055169019, ISSN: 0959-6658, DOI: 10.1093/glycob/cwr027
- TIMO RATH ET AL: "Fc-fusion proteins and FcRn: structural insights for longer-lasting and more effective therapeutics", CRITICAL REVIEWS IN BIOTECHNOLOGY, vol. 35, no. 2, 24 October 2013 (2013-10-24), US, pages 235 - 254, XP055597699, ISSN: 0738-8551, DOI: 10.3109/ 07388551.2013.834293
- MATHILDE BAS ET AL: "Fc Sialylation Prolongs Serum Half-Life of Therapeutic Antibodies", THE JOURNAL OF IMMUNOLOGY, vol. 202, no. 5, 1 March 2019 (2019-03-01), US, pages 1582 - 1594, XP055758310, ISSN: 0022-1767, DOI: 10.4049/ jimmunol.1800896
- PHILIPPE MONDON: "An innovative monomeric Fc fragment with high binding to FcRn and Fc gamma receptors for the treatment of autoimmune diseases", 1 June 2019 (2019-06-01), XP055758563, Retrieved from the Internet <URL:https://epo.summon. serialssolutions. com/2.0.0/link/0/eLvHCXMwrV07TwMxDI5ALDCB QOlpeWDkyl2SNpdKCKGKAhlTgoHplldz6tBrVQ ob_x376BU2Fi7LDZYVOUry2bE_C3EesaATQKU sBRUyLYPMHKGMLO_nwealK3xbFPY60s935ul OP26luquFwTbzDHvcO8rRpu9hxyd0Of-JY12vzPoQrziRMhA6q-gbDpPxyiFda2gHLlqmZi mLgEnaqPoey03aApzR07lPKXluLhd1T_KwWuu eMqb> [retrieved on 20201209], DOI: 10.13140/ rg.2.2.29444.37760
- MONNET C ET AL: "Combined glyco- and protein-Fc engineering simultaneously enhance cytotoxicity and half-life of a therapeutic antibody", MABS, LANDES BIOSCIENCE, US, vol. 6, no. 2, 1 March 2014 (2014-03-01), pages 422 - 436, XP002738427, ISSN: 1942-0862, [retrieved on 20140115], DOI: 10.4161/ MABS.27854
- CELINE MONNET ET AL: "Selection of IgG variants with increased FcRn binding using random and directed mutagenesis: impact on effector functions", FRONTIERS IN IMMUNOLOGY, vol. 6, no. 39, 4 February 2015 (2015-02-04), pages 1 - 14, XP055238838, DOI: 10.3389/fimmu.2015.00039

- WANG YUAN ET AL: "Neonatal Fc receptor (FcRn): a novel target for therapeutic antibodies and antibody engineering", JOURNAL OF DRUG TARGETING, HARWOOD ACADEMIC PUBLISHERS GMBH, DE, vol. 22, no. 4, 1 May 2014 (2014-05-01), pages 269 - 278, XP009185060, ISSN: 1061-186X, DOI: 10.3109/1061186X.2013.875030
- LUND J ET AL: "Multiple interactions of IgG with its core oligosaccharide can modulate recognition by complement and human Fc gamma receptor I and influence the synthesis of its oligosaccharide chains", THE JOURNAL OF IMMUNOLOGY, THE AMERICAN ASSOCIATION OF IMMUNOLOGISTS, US, vol. 157, no. 11, 1 December 1996 (1996-12-01), pages 4963 - 4969, XP002484003, ISSN: 0022-1767
- WARD E SALLY ET AL: "Chapter 4: Multitasking by exploitation of intracellular transport functions the many faces of FcRn", 1 January 2009, ADVANCES IN IMMUNOLOGY, ELSEVIER AMSTERDAM, NL, PAGE(S) 77 - 115, ISBN: 978-0-12-374832-4, XP008142597
- LAZAR G A ET AL: "Engineered antibody Fc variants with enhanced effector function", PROCEEDINGS OF THE NATIONAL ACADEMY OF SCIENCES, NATIONAL ACADEMY OF SCIENCES, US, vol. 103, no. 11, 1 March 2006 (2006-03-01), pages 4005 - 4010, XP002403708, ISSN: 0027-8424, DOI: 10.1073/PNAS.0508123103
- M. ALOULOU ET AL: "IgG1 and IVIg induce inhibitory ITAM signaling through Fc RIII controlling inflammatory responses", BLOOD, vol. 119, no. 13, 29 March 2012 (2012-03-29), US, pages 3084 - 3096, XP055311326, ISSN: 0006-4971, DOI: 10.1182/blood-2011-08-376046

Remarques:
Le document complet y compris le(s) table(s) de référence et le(s) listage(s) de séquence sont téléchargeables sur le site internet de l'OEB

**Description**

La présente invention concerne l'immunothérapie de maladies auto-immunes et/ou inflammatoires.

<u>**Arrière-plan technologique de l'invention**</u> :

L'immunothérapie, thérapeutique consistant à administrer à des patients des anticorps exogènes est aujourd'hui largement utilisée pour le traitement de diverses pathologies, particulièrement pour celui des maladies auto-immunes et des maladies inflammatoires.

Deux types de thérapie à base d'immunoglobulines sont typiquement proposés pour le traitement de ces maladies: i) les thérapies à base d'immunoglobulines intraveineuses (IgIV) qui consistent à administrer aux malades par voie intraveineuse des immunoglobulines (IgG le plus souvent) issues de pools de dons de plasma humain et ii) les thérapies qui se basent sur l'utilisation d'anticorps recombinants (à savoir des anticorps obtenus par génie génétique). Ces derniers ont permis de réelles avancées dans la prise en charge de patients atteints de maladies inflammatoires et de maladies auto-immunes, notamment parce qu'ils offrent la possibilité de s'affranchir des inconvénients liés à l'emploi d'immunoglobulines d'origine plasmatique, notamment le risque de rupture d'approvisionnement, et le risque de transmission de pathogènes éventuellement présents dans le plasma.

La présence des fragments Fab dans les immunoglobulines peut être à l'origine de la survenue d'importantes réactions indésirables chez les patients traités. Pour éviter ces effets secondaires, la demande de brevet WO 2004/099374 divulgue l'utilisation de fragments Fc recombinants isolés pour le traitement de patients, en particulier pour des patients atteints d'une maladie auto-immune.

La demande EP 2 233 500 décrit des fragments Fc ayant une affinité améliorée pour le FcRn par rapport à un fragment Fc parent, et comprenant au moins deux mutations.

Il existe cependant toujours un besoin d'optimiser ces fragments Fc, de manière notamment à augmenter leur temps de demi-vie, et/ou leur efficacité thérapeutique.

<u>**Résumé de l'invention**</u> :

Les inventeurs proposent maintenant d'utiliser des fragments Fc ayant une affinité modifiée pour au moins un des récepteurs Fc (FcR) par rapport à un fragment Fc parent. Selon l'invention, les fragments Fc sont isolés, à savoir qu'ils ne sont pas associés à des fragments Fab, ni conjugués ou fusionnés à toute autre protéine, polypeptide ou peptide. En particulier, il ne s'agit pas d'immunoglobulines complètes.

L'invention concerne une composition comprenant des fragments Fc d'anticorps, pour une utilisation dans le traitement d'une maladie spécifique telle que définie en revendication 1.

L'invention concerne également des compositions pour leur utilisation telles que définies dans l'une des revendications 2 à 8.

Selon des aspects préférés, il s'agit de

- fragments Fc présentent une combinaison de mutations K334N/P352S/A378V/V397M par rapport à un fragment Fc parent d'IgG1 sauvage, de préférence dans laquelle les fragments Fc consistent en la séquence SEQ ID NO :19.

L'invention fournit aussi plus généralement une composition comprenant des fragments Fc d'anticorps, pour une utilisation dans le traitement d'une maladie spécifique selon la revendication 1.

Dans un mode de réalisation particulier, la composition comprend des fragments Fc d'anticorps, comprenant au moins une mutation d'un ou plusieurs acides aminés et/ou possédant sur leur site de glycosylation (Asn 297) des N-glycannes, lesdits N-glycannes des fragments Fc présentant un taux de fucosylation inférieur à 65%,

<u>**Légende des figures**</u> :

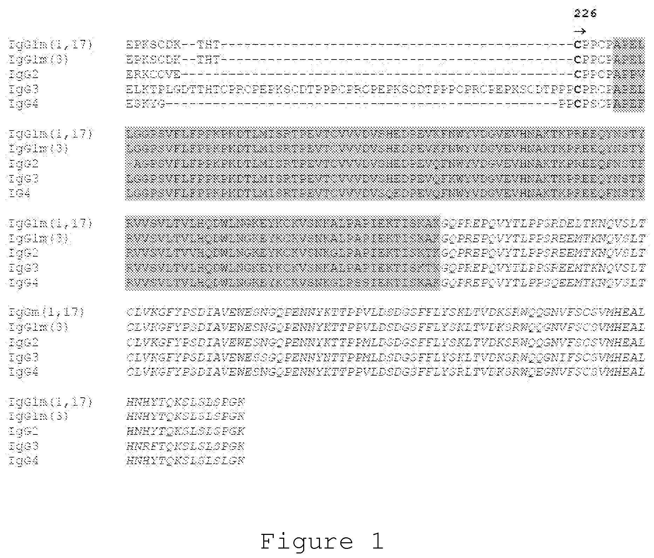

La **figure 1** montre des alignements de séquences d'IgG1 humaine natif se référant aux positions 216 à 447 (selon l'indice UE) avec les séquences correspondantes d'IgG2 humaine (SEQ ID NO: 7), IgG3 humaine (SEQ ID NO: 8) et IgG4 humaine (SEQ ID NO: 9). Les séquences d'IgG1 se réfèrent à l'allotype G1m1,17 (SEQ ID NO: 6) et à l'allotype G1m3 (SEQ ID NO: 10). Le domaine "charnière inférieure CH2-CH3" de l'IgG1 commence à la position 226 (voir flèche). Le domaine CH2 est surligné en gris et le domaine CH3 est en italique.

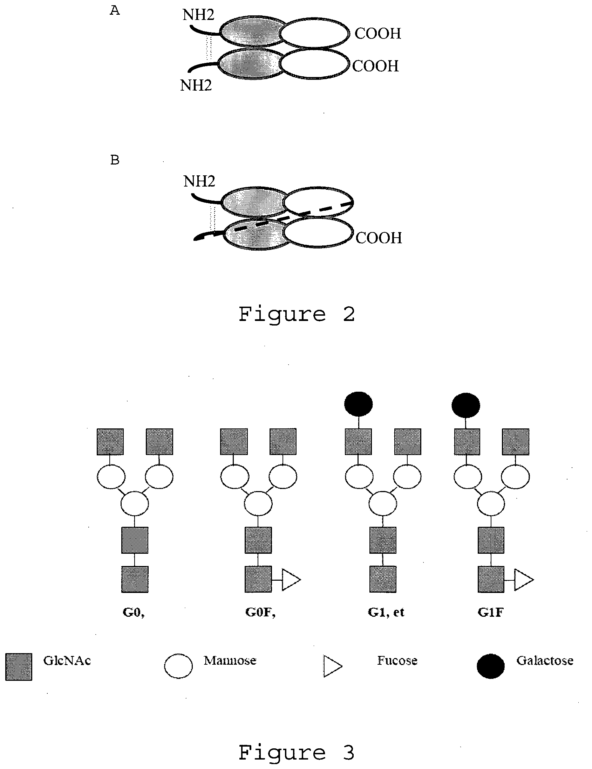

La **figure 2** montre des représentations schématiques du Fc IgG1 (A) et d'un scFc (B). Les CH2 sont représentés en gris et les CH3 en blanc. Les ponts disulfures sont représentés en pointillés gris fins. Dans le scFc, les deux polypeptides CH2-CH3 sont reliés par un linker peptidique représenté en pointillés gras.

La **figure 3** montre les formes G0, G0F, G1 et G1F des structures glycanniques susceptibles d'être présentes sur les fragments Fc de l'invention.

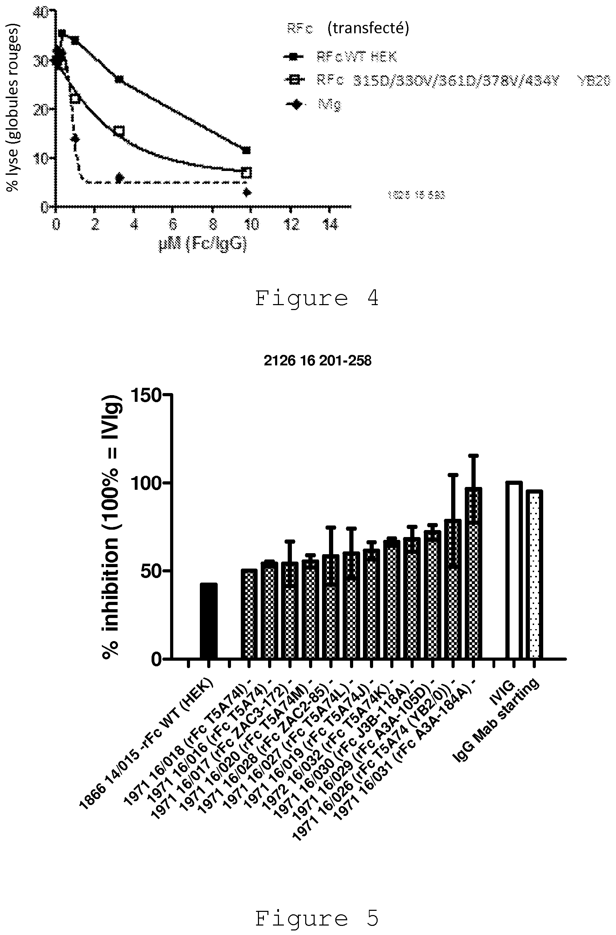

La **figure 4** est un graphe présentant le pourcentage de lyse de globules rouges Rhésus D+ médiée par des cellules effectrices en présence d'un anticorps monoclonal anti-Rhésus D (RhD), observée suite à l'addition de différentes quantités d'immunoglobulines polyvalentes (IgIV) ou de fragments Fc recombinants, non muté ou muté (fragment Fc portant les mutations 315D/330V/361D/378V/434Y).

La **figure 5** est un graphe présentant le pourcentage de lyse de globules rouges Rhésus D+ médiée par des cellules effectrices en présence d'un anticorps monoclonal anti-Rhésus D (RhD), observée suite à l'addition de différentes quantités d'immunoglobulines polyvalentes (IgIV) ou de fragments Fc recombinants, non muté ou muté (fragment Fc portant les mutations selon le **Tableau 1)**. rFc WT signifie le fragment Fc recombinant de type sauvage. Les contrôles positifs sont représentés par : IVIG (i.e. mélange d'anticorps plasmatiques extraits du plasma humain - les IVIG sont le traitement de référence du purpura thrombopénique idiopathique (PTI) et « IgG Mabs starting » qui correspond au fragment Fc de l'herceptin (anticorps produit par voie transgénique chez un mammifère, puis soumis à une digestion par la papaïne pour donner générer le fragment Fc).

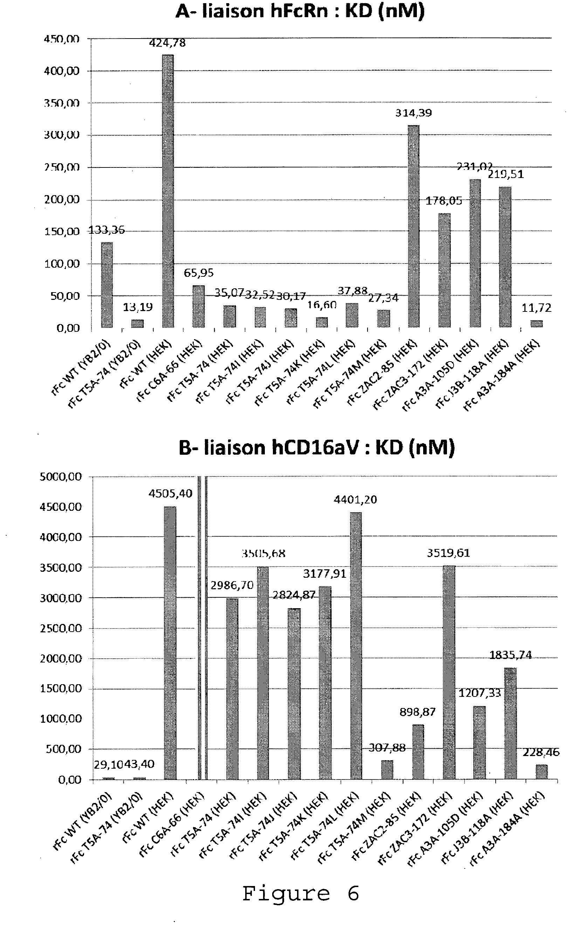

La **figure 6** représente un test de liaison in vitro mesurant l'affinité (Kd) des fragments Fc WT (de type sauvage) et mutés pour FcRn humain (A) et pour CD16aV (polymorphisme V en position 158 de CD16) (B), évaluée par la résonance plasmonique de surface (SPR) à l'aide du Biacore.

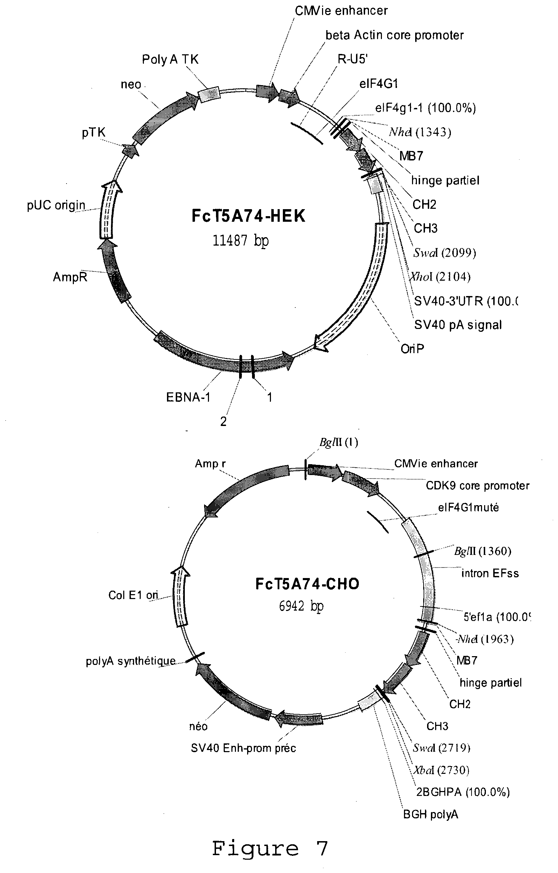

La **Figure 7** montre la carte du vecteur pCEP4 utilisé pour l'expression du variant T5A-74, défini plus bas, et la carte du vecteur OptiCHO pour l'expression du variant T5A-74.

## Description détaillée de l'invention :

### Définitions

Tout au long de la présente description, la numérotation des résidus dans le fragment Fc est celle de la chaîne lourde d'immunoglobuline selon l'indice EU ou tel que décrit dans Kabat et al., Séquences de protéines d'intérêt immunologique, 5e éd. Public Health Service, National Institutes of Health, Bethesda, Md. (1991), expressément incorporés ici par référence. «L'indice EU » ou « indice EU ou équivalent dans Kabat » se réfère ici à la numérotation des résidus de l'anticorps humain IgG1.

Le terme « immunoglobuline » se rapporte à la structure qui constitue la forme biologique à l'état naturel d'un anticorps, incluant les régions (également appelées fragments) constantes et variables. Une molécule d'immunoglobuline est une molécule dont l'unité de base est un hétérotétramère constitué de deux chaînes lourdes d'environ 50-70 kDa chacune (dites les chaînes H pour Heavy) et de deux chaînes légères d'environ 25 kDa chacune (dites les chaînes L pour Light), liées entre elles par des ponts disulfures intra et intercaténaires.

Chaque chaîne est constituée, en position N-terminale, d'une région ou domaine variable, dit VL pour la chaîne légère, VH pour la chaîne lourde, et en position C-terminale, d'une région constante, constitué d'un seul domaine dit CL pour la chaîne légère et de trois ou quatre domaines nommés CH1, CH2, CH3, CH4, pour la chaîne lourde.

Seules les IgM et les IgE possèdent le domaine CH4.

Chaque domaine comprend environ 110 acides aminés et est structuré de manière comparable. Les 2 chaînes lourdes sont liées par des ponts disulfures au niveau des CH2 et chaque chaîne lourde est liée à une chaîne légère par un pont disulfure entre le CH1 et le CL. La région qui détermine la spécificité de l'anticorps pour l'antigène est portée par les parties variables, alors que les parties constantes peuvent interagir avec les récepteurs Fc (RFc) des cellules effectrices ou des molécules comme les protéines du complément pour induire différentes propriétés fonctionnelles. L'assemblage des chaînes qui composent un anticorps permet de définir une structure tridimensionnelle caractéristique en Y, où

- la base du Y correspond à la région constante Fc ; ou fragment Fc ; qui est reconnue par le complément et les récepteurs Fc afin de médier les fonctions effectrices de la molécule, et
- les extrémités des bras du Y correspondent à l'assemblage respectif de la région variable d'une chaîne légère et de la région variable d'une chaîne lourde, lesdites extrémités constituent le fragment Fab et déterminent la spécificité de l'anticorps pour l'antigène.

Plus précisément, il existe cinq isotypes de chaînes lourdes (gamma, alpha, mu, delta et epsilon) et deux isotypes de chaînes légères (kappa et lambda, les chaînes lambda étant elles-mêmes divisées en deux types : lambda 1 et lambda 2). C'est la chaîne lourde qui détermine la classe d'immunoglobuline. Il y a ainsi cinq classes d'Ig : IgG pour l'isotype Gamma, IgA pour l'isotype Alpha, IgM pour l'isotype Mu, IgD pour l'isotype Delta et IgE pour l'isotype Epsilon.

Les chaînes légères kappa et lambda sont partagées par toutes les classes et sous-classes. Chez l'homme, la proportion de kappa et lambda produite se situe dans un rapport de 2 pour 1.

Les Immunoglobulines G (IgG) sont les immunoglobulines les plus abondantes dans le sérum (75 à 80% des anticorps circulants). Présentes sous forme de monomères, elles présentent la plus longue demi-vie sérique de toutes les immunoglobulines (environ 21 jours).

Il existe quatre types de chaînes lourdes gamma, ce qui détermine quatre sous-classes d'IgG (IgG1 pour gamma1, IgG2 pour gamma2, IgG3 pour gamma3 et IgG4 pour gamma4). Ces quatre sous-classes diffèrent par des nombres et des positions variables des ponts disulfures (Basic and Clinical Immunology, 8ème édition, Daniel P. Stites, Abba I. Terr and Tristram G. Parslow (eds.), Appleton & Lange, Norwalk, Conn., 1994, page 71 et Chapitre 6).

Les quatre sous-classes des IgG humaines se distinguent également au niveau de leurs activités biologiques, malgré des structures très homologues (plus de 95% d'homologie de séquence pour les régions Fc).

Par activité biologique, on fait notamment référence à la capacité de la région constante des IgG à lier : notamment les protéines du complément (la protéine C1q par exemple [Basic and Clinical Immunology, 8ème édition, Daniel P. Stites, Abba I. Terr and Tristram G. Parslow (eds.)]) et/ou les récepteurs aux IgG : RFcγ (RFcγI, RFcγII, RFcγIII, Ravetch and Kinet, AnnualReview of Immunology, vol 9:457-492 (1991)).

En fonction du type de liaison, divers mécanismes d'action pourront être activés : l'opsonisation, la phagocytose, l'ADCC (Antibody Dépendant Cellular Cytotoxicity) ou la CDC (Complément Dépendant Cytotoxicity) par exemple. Voir Uananue and Benacerraf, Textbook of Immunology, 2nd Edition, Williams & Wilkins, p. 218 (1984)) pour plus de détails.

Par " fragment Fc ", on entend la région constante d'une immunoglobuline de longueur totale à l'exclusion du premier domaine de région constante d'immunoglobuline. Ainsi le fragment Fc fait référence aux deux derniers domaines constants (CH2-CH3) des IgA, IgD, IgG et les trois derniers domaines constants (CH2-CH3-CH4) des IgE et IgM, et la charnière flexible N-terminale de ces domaines. Pour les IgA et IgM, le fragment Fc peut comprendre la chaîne J. Pour les IgG, le fragment Fc comprend les domaines CH2, CH3 et la région charnière inférieure entre CH1 et CH2. En d'autres termes, la région Fc d'une IgG1 se compose de la charnière inférieure CH2-CH3, c'est-à-dire la portion à partir de l'acide aminé C226 jusqu'à l'extrémité carboxy-terminale, la numérotation étant indiquée selon l'indice EU ou équivalent dans Kabat. Les domaines analogues pour d'autres sous-classes d'IgG peuvent être déterminés à partir de l'alignement des séquences d'acides aminés des chaînes lourdes ou des fragments de chaînes lourdes des sous-classes d'IgG avec celle d'une IgG1 humaine (cf Figure 1). Le fragment Fc utilisé selon l'invention peut comprendre en outre une partie de la région charnière supérieure, en amont de la position 226 (selon l'indice EU). Dans ce cas, de préférence, on utilise un fragment Fc d'une IgG1 humaine comprenant une partie de la région située entre les positions 216 à 226. Dans ce cas, le fragment Fc d'une IgG1 humaine fait référence à la portion à partir de l'acide aminé 216, 217, 218, 219, 220, 221, 222, 223, 224, ou 225 jusqu'à l'extrémité carboxy-terminale. Par « fragment Fc », on entend également un fragment scFc pour « single chain Fc ». Par « fragment scFc », on entend un fragment Fc simple chaîne, obtenu par fusion génétique de deux monomères Fc reliés par un linker polypeptidique. Le scFc se replie naturellement en une région Fc dimérique fonctionnelle.

Par « fragment Fc parent » ou « polypeptide parent » tel qu'utilisé ici, on entend un polypeptide de référence parmi les régions Fc de type sauvage ou des variants portant éventuellement d'autres mutations que celle considérée. Par " type sauvage ou WT", on entend ici une séquence d'acides aminés ou une séquence nucléotidique que l'on trouve dans la nature c'est à dire qui est d'origine naturelle, y compris des variations alléliques, et qui n'a pas été intentionnellement modifiée par des techniques de biologie moléculaire telles que la mutagenèse. Par exemple, les régions Fc « de type sauvage » se réfèrent notamment à la région Fc de l'IgG1 ayant la séquence SEQ ID N°1 (allotype G1m1,17), la région Fc d' IgG2 ayant la séquence SEQ ID N ° 2, la région Fc d' IgG3 ayant la séquence SEQ ID N ° 3, la région Fc d' IgG4 ayant la séquence SEQ ID N ° 4, et la région Fc d' IgG1 ayant la séquence SEQ ID N ° 5 (allotype G1m3).

Les fragments Fc utilisés dans l'invention sont sous forme monomère, à savoir qu'ils ne sont pas fusionnés ni conjugués entre eux.

Par « affinité modifiée » on entend une affinité diminuée ou augmentée par rapport à un fragment Fc parent.

Par " FcRn " ou " récepteur Fc néonatal ", tel qu'utilisé ici, on entend une protéine qui se lie à la région Fc des IgG et est codée au moins en partie par un gène FcRn. Le FcRn peut être de n'importe quel organisme, y compris, mais sans s'y limiter, les humains, les souris, les rats, les lapins et les singes. Comme cela est connu dans la technique, la protéine FcRn fonctionnelle comprend deux polypeptides, souvent désignés sous le nom de chaîne lourde et de chaîne légère. La chaîne légère est la bêta -2 -microglobuline et la chaîne lourde est codée par le gène FcRn. Sauf indication contraire ici, FcRn ou protéine FcRn se réfère au complexe de la chaîne α avec la bêta- 2-microglobuline. Chez l'homme, le gène codant pour le FcRn est appelé FCGRT.

Par "augmentation de la liaison au FcRn " tel qu'il est utilisé ici, on entend l'augmentation de l'affinité de liaison, *in vivo* ou *in vitro,* du fragment Fc muté pour le FcRn, par rapport au polypeptide parent. La capacité du fragment Fc muté à se lier à un récepteur FcRn peut être évaluée *in vitro* par un test ELISA, comme décrit par exemple dans la demande de brevet WO2010/106180.

Le terme « demi-vie » désigne, dans la présente invention, le temps que met le fragment Fc pour être éliminé pour moitié de la circulation ou d'autres tissus, une fois présent dans le sérum du patient auquel il a été administré.

Le terme « Récepteur Fcγ » ou « FcγR » se réfère aux récepteurs des Immunoglobulines de type IgG, appelés CD64 (FcγRI), CD32 (FcγRII), et CD16 (FcγRIII), en particulier cinq récepteurs exprimés (FcγRIa, FcγRIIa, FcγRIIb,

FcγRIIIa, FcγRIIIb). Tous sont des récepteurs activateurs des cellules effectrices, hormis le FcγRIIb humain qui est un récepteur inhibiteur de l'activation des cellules immunitaires (Muta T et al., *Nature*, 1994, 368 :70-73).

Par « cellule effectrice », on entend toute cellule porteuse d'un récepteur Fc, telles que les lymphocytes, les monocytes, les neutrophiles, les cellules Natural Killer (NK), les éosinophiles, les basophiles, les mastocytes, les cellules dendritiques, les cellules de Langerhans et les plaquettes.

Le terme « glycosylation », dans le contexte de l'invention, se réfère à l'ajout, par réaction enzymatique, d'un ou plusieurs glucides à la séquence d'un fragment Fc recombinant.

Le terme « hypersialysation », dans le contexte de l'invention, se réfère à l'ajout d'un ou plusieurs groupements d'acide sialique à la séquence d'un fragment Fc. L'ajout d'un ou plusieurs groupes d'acide sialique peut être réalisé par réaction enzymatique, réaction cellulaire ou par mutagenèse dirigée du Fc ciblant un ou plusieurs acides aminés impliqués dans la sialylation du Fc.

Le terme « patient » se réfère à tout sujet humain ou animal, de préférence mammifère. Dans un mode de réalisation préféré, le patient est un être humain, quel que soit son âge et son sexe.

Le terme " traitement " ou " traiter " désigne une amélioration, la prophylaxie, ou l'inversion d'une maladie ou d'un trouble, ou au moins d'un symptôme pouvant être discerné de celui-ci, ou une amélioration, la prophylaxie, ou l'inversion d'au moins un paramètre physique mesurable associé à la maladie ou au trouble étant traité, qui n'est pas nécessairement discernable chez ou par le sujet traité. Le terme "traitement " ou " traiter " comprend encore l'inhibition ou le ralentissement de la progression d'une maladie ou un trouble, physiquement, par exemple, la stabilisation d'un symptôme discernable, physiologiquement, par exemple, la stabilisation d'un paramètre physique, ou les deux.

### Compositions de fragments Fc selon l'invention

Les fragments Fc d'anticorps utilisés dans l'invention sont des fragments Fc d'une immunoglobuline IgG1, ayant une affinité modifiée pour au moins un des récepteurs Fc (FcR) par rapport à un fragment Fc parent.

Les fragments Fc selon l'invention présentent une affinité augmentée à au moins un récepteur Fc, par rapport à un fragment Fc parent.

L'affinité est augmentée, par rapport à celle du Fc parent, d'un ratio au moins égal à 2, de préférence supérieur à 5, de préférence supérieur à 10, de préférence supérieur à 15, de préférence supérieur à 20, de préférence supérieur à 25, et de préférence supérieur à 30. En d'autres termes, l'affinité de la région Fc mutée pour un FcR est supérieure à celle du polypeptide parent.

L'affinité d'un polypeptide comprenant une région Fc pour un FcR peut être évaluée par des procédés bien connus de l'art antérieur. Par exemple, l'homme de l'art peut déterminer l'affinité (Kd) en utilisant la résonance plasmonique de surface (SPR) qui peut être mesuré par l'appareil Biacore. Alternativement, l'homme de l'art peut effectuer un test ELISA approprié. Un dosage ELISA approprié permet de comparer les forces de liaison du Fc parent et du Fc muté. Les signaux détectés spécifiques du Fc muté et du Fc parent sont comparés. L'affinité de liaison peut être indifféremment déterminée en évaluant les polypeptides entiers ou en évaluant les régions Fc isolées de ceux-ci.

Selon un mode de réalisation particulier de la divulgation, les fragments Fc utilisés selon l'invention présentent une affinité modifiée, avantageusement augmentée, au récepteur FcRn. Cette augmentation de la liaison au FcRn se traduit par une amélioration de la rétention de sérum *in vivo* et, par conséquent, une augmentation de la demi-vie.

Les fragments Fc peuvent être utilisés seuls ou en mélange, par exemple plusieurs fragments Fc, présentant des mutations différentes peuvent être administrés en mélange ou co-administrés.

Les fragments Fc de l'invention peuvent également être mis en oeuvre dans une composition comprenant un seul type de fragment Fc muté. En d'autres termes, la composition comprend alors des molécules de fragments Fc de séquence identique.

Dans un mode de réalisation de la divulgation, les fragments Fc portent une combinaison de mutations choisie parmi 315D/330V/361D/378V/434Y (combinaison de mutations également dénommée « T5A-74 ». Les fragments Fc portant cette combinaison de mutation sont donc également appelés « rFc T5A-74), 230S/315D/428L/434Y, 307A/315D/330V/382V/389T/434Y, 259I/315D/434Y, 256N/378V/383N/434Y, E294del/T307P/N434Y (combinaison de mutations également dénommée « C6A_66 ». Les fragments Fc portant cette combinaison de mutation sont donc également appelés « rFc C6A_66).

Dans un mode de réalisation de la divulgation, les fragments Fc portent une combinaison de mutations choisie parmi :

(i) T260A et 315D/330V/361D/378V/434Y (combinaison de mutations également dénommée « T5A-74 »)- Le fragment désigné « T5A-74I » est un fragment Fc qui ne se distingue du fragment T5A-74 que par l'ajout de la mutation T260A.
Ou

(ii) E258I et 315D/330V/361D/378V/434Y (combinaison de mutations également dénommée « T5A-74 »)- Le fragment désigné « T5A-74J » est un fragment Fc qui ne se distingue du fragment T5A-74 que par l'ajout de la mutation E258I.
Ou

(iii) K290Y et 315D/330V/361D/378V/434Y (combinaison de mutations également dénommée « T5A-74 ») - Le fragment désigné « T5A-74K » est un fragment Fc qui ne se distingue du fragment T5A-74 que par l'ajout de la mutation K290Y.
Ou

(iv) E294A et 315D/330V/361D/378V/434Y (combinaison de mutations également dénommée « T5A-74 »)- Le fragment désigné « T5A-74L » est un fragment Fc qui ne se distingue du fragment T5A-74 que par l'ajout de la mutation E294A.
Ou

(v) Y296W et 315D/330V/361D/378V/434Y (combinaison de mutations également dénommés « T5A-74 »)- Le fragment désigné « T5A-74M » est un fragment Fc qui ne se distingue du fragment T5A-74 que par l'ajout de la mutation Y296W.

Les séquences des fragments Fc T5A-74, T5A-74I, T5A-74J, T5A-74K, T5A-74L, T5A-74M, sont présentées dans le listing de séquence joint.

T5A-74 (N315D/A330V/N361D/A378V/N434Y) :

SEQ ID NO : 11

DKTHTCPPCPAPELLGGPSVFLFPPKPKDTLMISRTPEVTCVVVDVSHEDPEVKFNWY

VDGVEVHNAKTKPREEQYNSTYRVVSVLTVLHQDWLDGKEYKCKVSNKALPVPIEK

TISKAKGQPREPQVYTLPPSRDELTKDQVSLTCLVKGFYPSDIVVEWESNGQPENNYK

TTPPVLDSDGSFFLYSKLTVDKSRWQQGNVFSCSVMHEALHYHYTQKSLSLSPGK

T5A-74I (T260A/N315D/A330V/N361D/A378V/N434Y)

SEQ ID NO : 12

DKTHTCPPCPAPELLGGPSVFLFPPKPKDTLMISRTPEVACVVVDVSHEDPEVKFNWY

VDGVEVHNAKTKPREEQYNSTYRVVSVLTVLHQDWLDGKEYKCKVSNKALPVPIEK

TISKAKGQPREPQVYTLPPSRDELTKDQVSLTCLVKGFYPSDIVVEWESNGQPENNYK

TTPPVLDSDGSFFLYSKLTVDKSRWQQGNVFSCSVMHEALHYHYTQKSLSLSPGK

T5A-74J (E258I/N315D/A330VIN361D/A378VIN434Y)

SEQ ID NO : 13

DKTHTCPPCPAPELLGGPSVFLFPPKPKDTLMISRTPIVTCVVVDVSHEDPEVKFNWY

VDGVEVHNAKTKPREEQYNSTYRVVSVLTVLHQDWLDGKEYKCKVSNKALPVPIEK

TISKAKGQPREPQVYTLPPSRDELTKDQVSLTCLVKGFYPSDIVVEWESNGQPENNYK

TTPPVLDSDGSFFLYSKLTVDKSRWQQGNVFSCSVMHEALHYHYTQKSLSLSPGK

T5A-74K (K290Y/N315D/A330V/N361D/A378V/N434Y)

SEQ ID NO : 14

DKTHTCPPCPAPELLGGPSVFLFPPKPKDTLMISRTPEVTCVVVDVSHEDPEVKFNWY
VDGVEVHNAKTYPREEQYNSTYRVVSVLTVLHQDWLDGKEYKCKVSNKALPVPIEK
TISKAKGQPREPQVYTLPPSRDELTKDQVSLTCLVKGFYPSDIVVEWESNGQPENNYK
TTPPVLDSDGSFFLYSKLTVDKSRWQQGNVFSCSVMHEALHYHYTQKSLSLSPGK

T5A-74L (E294A/N315D/A330V/N361D/A378V/N434Y)

SEQ ID NO : 15

DKTHTCPPCPAPELLGGPSVFLFPPKPKDTLMISRTPEVTCVVVDVSHEDPEVKFNWY
VDGVEVHNAKTKPREAQYNSTYRVVSVLTVLHQDWLDGKEYKCKVSNKALPVPIE
KTISKAKGQPREPQVYTLPPSRDELTKDQVSLTCLVKGFYPSDIVVEWESNGQPENNY
KTTPPVLDSDGSFFLYSKLTVDKSRWQQGNVFSCSVMHEALHYHYTQKSLSLSPGK

T5A-74M (Y296W/N315D/A330V/N361D/A378V/N434Y)

SEQ ID NO : 16

DKTHTCPPCPAPELLGGPSVFLFPPKPKDTLMISRTPEVTCVVVDVSHEDPEVKFNWY
VDGVEVHNAKTKPREEQWNSTYRVVSVLTVLHQDWLDGKEYKCKVSNKALPVPIE
KTISKAKGQPREPQVYTLPPSRDELTKDQVSLTCLVKGFYPSDIVVEWESNGQPENNY
KTTPPVLDSDGSFFLYSKLTVDKSRWQQGNVFSCSVMHEALHYHYTQKSLSLSPGK

La divulgation décrit également des fragments Fc mutés qui ont une affinité augmentée pour le récepteur FcγRIIIa (CD16a), et comprennent au moins une combinaison de 2 mutations, ladite combinaison comprenant:

i) une mutation choisie parmi 378V, 326E, 397M, 334N et 396L ; et
ii) au moins une mutation choisie parmi 316D, 397M, 334N, 248E, 231V, 246R, 336T, 421T, 361H, 366A, 439R, 290E, 394P, 307P, 378V, 378T, 286I, 286Y et 298N,

la numérotation étant celle de l'index EU ou équivalent dans Kabat et avec la condition que la mutation (i) n'a pas lieu sur le même acide aminé que la mutation (ii).

La divulgation décrit également des fragments Fc mutés comprenant au moins une combinaison de 3 mutations, ladite combinaison comprenant :

(i) une mutation choisie parmi 326E, 326T, 352L, 378V, 378T, 396L, 397M, 421T, 334N, 334R, 307N et 394P ; et
(ii) au moins 2 mutations choisies parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N,

la numérotation étant celle de l'index EU ou équivalent dans Kabat et avec la condition que la mutation (i) n'a pas lieu sur le même acide aminé que la mutation (ii).

Les fragments Fc de l'invention portent la combinaison de mutations K334N/P352S/V397M/A378V (combinaison de mutations également dénommée « A3A-184A »). Les fragments Fc portant cette combinaison de mutations sont donc également appelés « A3A-184A ).

La séquence du fragment A3A-184A (K334N/P352S/A378V/V397M) est présentée dans le listing de séquences, comme SEQ ID NO : 19 :

DKTHTCPPCPAPELLGGPSVFLFPPKPKDTLMISRTPEVTCVVVDVSHEDPEVKFNWY

VDGVEVHNAKTKPREEQYNSTYRVVSVLTVLHQDWLNGKEYKCKVSNKALPAPIEN

TISKAKGQPREPQVYTLSPSRDELTKNQVSLTCLVKGFYPSDIVVEWESNGQPENNYK

TTPPMLDSDGSFFLYSKLTVDKSRWQQGNVFSCSVMHEALHNHYTQKSLSLSPGK

Selon un autre mode de réalisation de la divulgation, les fragments Fc mutés portent une délétion d'un acide aminé en position 293 ou 294 (DEL293 ou DEL294), la numérotation des acides aminés du fragment Fc se référant à celle de l'indice EU, ou l'équivalent dans Kabat.

Cette seule mutation conduit à une glycosylation particulière du fragment, à savoir une hypersialylation, particulièrement avantageuse vis-à-vis de la demi-vie des glycoprotéines et des processus inflammatoires.

Selon un autre aspect de l'invention, on utilise une composition comprenant une pluralité de fragments Fc qui possèdent tous substantiellement la même séquence, et qui, pris dans leur ensemble, présentent un profil de glycosylation particulier.

Selon un aspect particulier, une composition utilisée dans le cadre de l'invention comprend des fragments Fc possédant sur leur site de glycosylation (Asn 297) des N-glycannes, caractérisée en ce que lesdits N-glycannes des fragments Fc présentent un taux de fucosylation inférieur à 65%, de préférence inférieur à 60%, de préférence inférieur à 55%, de préférence inférieur à 50%, de préférence encore inférieur à 45%, de préférence inférieur à 40%, de préférence inférieur à 35%, de préférence inférieur à 30%, de préférence inférieur à 25%, de préférence inférieur à 20%.

Selon un autre aspect encore, une composition utilisée dans le cadre de l'invention comprend des fragments Fc, lesdits fragments Fc possédant sur leur site de glycosylation (Asn 297) des N-glycannes, caractérisée en ce que lesdits N-glycannes des fragments Fc présentent une structure glycannique de type biantenné, avec des chaînes courtes, une faible sialylation, présentant des mannoses terminaux et/ou des N-acétylglucosamines terminaux non intercalaires.

Selon un aspect plus particulier, une composition utilisée dans le cadre de l'invention comprend des fragments Fc, lesdits fragments Fc possédant sur leur site de glycosylation (Asn 297) des N-glycannes, caractérisée en ce que lesdits N-glycannes des fragments Fc présentent une teneur supérieure à 60 % pour les formes G0+G1+G0F+G1F, les formes G0F+G1F étant inférieures à 50 %.

Selon un autre aspect plus particulier une composition utilisée dans le cadre de l'invention comprend des fragments Fc, lesdits fragments Fc possédant sur leur site de glycosylation (Asn 297) des N-glycannes, caractérisée en ce que lesdits N-glycannes des fragments Fc présentent une teneur supérieure à 60 % pour les formes G0+G1+G0F+G1F, la teneur en fucose étant inférieure à 65 %.

Selon un autre aspect encore plus particulier une composition utilisée dans le cadre de l'invention comprend des fragments Fc, lesdits fragments Fc possédant sur leur site de glycosylation (Asn 297) des N-glycannes, caractérisée en ce que lesdits N-glycannes des fragments Fc présentent une teneur inférieure à 40% pour les formes G1F+GOF.

Selon un aspect plus particulier, une composition utilisée dans le cadre de l'invention comprend des fragments Fc qui possèdent sur leur site de glycosylation (Asn 297) des N-glycannes, lesdits N-glycannes des fragments Fc présentant un taux de fucosylation égal à 0%. L'invention fournit ainsi une composition comprenant des fragments Fc qui possèdent sur le site de glycosylation Asn297 des N-glycannes caractérisée en ce que lesdits N-glycannes des fragments Fc sont dépourvus de fucose.

Aussi, selon un aspect particulier, une composition utilisée dans le cadre de l'invention comprend des fragments Fc qui possèdent sur le site de glycosylation Asn297 des N-glycannes, caractérisée en ce que lesdits N-glycannes des fragments Fc présentent un taux de fucosylation compris entre 20 % et 55%. En particulier, l'invention fournit une composition comprenant des fragments Fc qui possèdent sur le site de glycosylation Asn297 des N-glycannes caractérisée en ce que lesdits N-glycannes des fragments Fc présentent un taux de fucosylation compris entre 20 % et 50%, entre 25% et 55%, entre 25% et 50%, entre 20% et 45% ou entre 25 et 45%.

Selon un aspect plus particulier, une composition utile selon l'invention comprend des fragments Fc qui possèdent sur le site de glycosylation Asn297 des N-glycannes, caractérisée en ce que lesdits N-glycannes des fragments Fc présentent une teneur supérieure à 60 %, de préférence supérieure à 80%, pour les formes G0+G1+G0F+G1F, les formes G0F+G1F étant inférieures à 50 %, de préférence inférieure à 40%, ou à 30%.

Selon un autre aspect plus particulier, les N-glycannes des fragments Fc au sein de la composition présentent une teneur supérieure à 60 % pour les formes G0+G1+G0F+G1F, la teneur en fucose étant inférieure à 65 %.

Selon encore un autre aspect plus particulier, les N-glycannes des fragments Fc au sein de la composition présentent une teneur inférieure à 50% pour les formes G1F+GOF, de préférence inférieure à 40%, ou à 30%.

Les formes G0, G0F, G1 et G1F sont sélectionnées parmi les formes indiquées sur la Figure 3.

De manière avantageuse, les N-glycannes des fragments Fc au sein de la composition ont une teneur moyenne en acide sialique inférieure à 25%, 20%, 15%, ou 10%, de préférence 5%, 4% 3% ou 2%.

Une composition qui peut être utilisée dans le cadre de l'invention comprend des fragments Fc possédant sur leur site de glycosylation (Asn 297) des N-glycannes, lesdits N-glycannes des fragments Fc présentant une structure glycannique de type biantenné, avec des chaînes courtes, une faible sialylation, présentant des mannoses terminaux et/ou des N-acétylglucosamines terminaux non intercalaires, les N-glycannes ayant par exemple une teneur supérieure à 60% pour les formes G0 + G1 + G0F + G1F, et une faible fucosylation, les N-glycannes ayant par exemple une teneur inférieure a 50% de formes G0F + G1F.

Dans un mode de réalisation particulier, les fragments Fc selon l'invention présentent des structures glycaniques telles que décrites dans la demande de brevet WO01/77181.

Selon un mode de réalisation avantageux, les fragments Fc utilisés dans l'invention comprennent au moins la séquence SEQ ID NO :19, et possèdent sur leur site de glycosylation (Asn 297) des N-glycannes, lesdits N-glycannes des fragments Fc présentant un taux de fucosylation inférieur à 65%, de préférence inférieur à 60%, de préférence inférieur à 55%, de préférence inférieur à 50%, de préférence encore inférieur à 45%, de préférence inférieur à 40%, de préférence inférieur à 35%, de préférence inférieur à 30%, de préférence inférieur à 25%, de préférence inférieur à 20%.

De façon avantageuse, les fragments Fc ayant une glycosylation modifiée au niveau du site de glycosylation en position 297, en particulier une faible fucosylation, présente une augmentation de la liaison dudit fragment aux récepteurs Fcgamma (FcyR), en particulier le récepteur FcyRIIIa (CD16a).

De préférence, lesdits fragments Fc ont une affinité pour le CD16a au moins égale à $2x10^6$ $M^{-1}$ au moins égale à $2x10^7$ $M^{-1}$, $2x10^8$ $M^{-1}$ ou $2x10^9$ $M^{-1}$, telle que déterminée par l'analyse Scatchard ou la technologie BIAcore (Label-free surface plasmon résonance based technology).

De façon particulièrement avantageuse, les fragments Fc mutés de l'invention peuvent être mis en oeuvre en combinaison de différents fragments Fc mutés portant des mutations différentes. Par exemple, on peut utiliser de manière avantageuse un mélange de Fc mutés Fc-De1294, Fc mutés T5A-74 (pour cibler aussi FcRn), Fc mutés améliorés pour la liaison aux FcyRs (types A3A-184A).

### Production des fragments Fc selon l'invention

Les fragments Fc utilisés dans l'invention peuvent être produits par toute méthode connue de l'homme du métier, par exemple par voie de synthèse chimique, ou par voie de recombinaison.

Dans un mode de réalisation préféré, les fragments Fc utilisés dans l'invention sont dits « recombinants », à savoir qu'ils sont obtenus par voie de recombinaison.

Lorsque les fragments Fc selon l'invention comprennent une mutation d'un ou plusieurs acides aminés, la ou les mutations peuvent être éventuellement introduites par des techniques connues telles qu'une synthèse de gène, une mutagenèse dirigée, notamment obtenue par PCR avec des amorces spécifiques qui introduisent les mutations souhaitées, la mutagenèse aléatoire. De préférence, la mutagénèse aléatoire telle que décrite dans la demande WO02/038756 est utilisée : il s'agit de la technique MutaGen. Cette technique utilise une ADN mutase humaine, notamment choisie parmi les ADN polymérases β, η et ι. Les techniques recombinantes classiques mettent en oeuvre une recombinaison dans une cellule hôte, transformée avec un ou des vecteur(s) qui permettent l'expression accompagnée ou non de la sécrétion de la séquence du fragment Fc dans le milieu extracellulaire. Le vecteur comporte généralement un promoteur, des signaux d'initiation et de terminaison de la traduction, ainsi que des régions appropriées de régulation de la transcription. Il peut être maintenu de façon stable dans la cellule hôte et peut éventuellement posséder des signaux particuliers qui spécifient la sécrétion de la protéine traduite. Ces différents éléments sont choisis et optimisés par l'homme du métier en fonction de l'hôte cellulaire utilisé.

De tels vecteurs sont préparés par des méthodes couramment utilisées par l'homme du métier, et les clones résultant peuvent être introduits dans un hôte approprié par des méthodes standard, telles que la lipofection, l'électroporation, l'utilisation d'agents polycationiques, le choc thermique, ou des méthodes chimiques.

L'hôte cellulaire peut être choisi parmi des systèmes procaryotes ou eucaryotes, par exemple les cellules bactériennes mais également les cellules de levure ou les cellules animales, en particulier les cellules de mammifères. On peut également utiliser des cellules d'insectes ou des cellules de plantes.

Les fragments Fc utilisés dans l'invention peuvent être produits par mise en culture dans un milieu et conditions de culture appropriés d'une cellule hôte exprimant lesdits fragments Fc; et récupération des fragments ainsi produits à partir du milieu de culture ou desdites cellules cultivées.

Les cellules de mammifère préférées pour l'expression des fragments Fc sont la lignée de rat YB2/0, la lignée vero, la lignée de hamster CHO, en particulier les lignées CHO dhfr- et CHO Lec13, CHO-lec10, CHO-lec1, CHOK1SV Potelligent® (Lonza, Suisse), CHOGnTIII (Glycart, Suisse), PER.C6™ (Crucell), HEK293, T1080, EB66, K562, NS0, SP2/0, BHK ou COS.

Un autre mode de production est l'expression des fragments Fc dans des organismes transgéniques, par exemple dans les plantes (Ayala M, Gavilondo J, Rodriguez M, Fuentes A, Enríquez G, Pérez L, Cremata J, Pujol M. Production of plantibodies in Nicotiana plants. Methods Mol Biol. 2009;483:103-34.) ou bien dans le lait d'animaux transgéniques tels que le lapin, la chèvre, le rat ou le porc (Pollock, D.P., J.P. Kutzko, E. Birck-Wilson, J.L. Williams, Y. Echelard and H.M. Meade. (1999). Transgenic milk as a method for the production of recombinant antibodies. Journal of Immunological Methods. 231: 147-157). Voir également le document WO200748077 à cet égard.

Dans un mode de réalisation alternatif, les fragments Fc sont obtenus par traitement protéolytique d'immunoglobulines elles-mêmes mutées.

La modification de la glycosylation des fragments Fc peut être obtenue par des techniques connues. Les fragments Fc possédant une glycosylation selon l'invention peuvent notamment être produits à partir du clivage d'anticorps produits selon la technique décrite dans WO 01/77181, notamment par l'enzyme papaïne. Les fragments Fc faiblement fucosylés peuvent être aussi obtenus par production dans des cellules cultivées en présence de kifunensine, comme cela est décrit par exemple dans le document US7700321, ou dans des cellules pour lesquelles la voie de production des GDP-fucose est inhibée, par exemple par inhibition de l'une au moins des enzymes du cycle de production fucose (cf notamment les documents US 2010291628 ou US 20090228994, EP 1 500 698, EP 1 792 987 ou US 7 846 725). Il est également possible d'utiliser des ARN interférents (ARNi) inhibant la 1,6-fucosyltransférase comme décrit dans le document US 7 393 683 ou le document WO2006133148. Il peut également s'agir de méthodes de préparation dans des levures, comme cela est décrit par exemple dans le document WO 0200879.

Dans le cas où les fragments Fc possèdent 100% d'oligosaccharides non fucosylés, c'est-à-dire quand les fragments Fc sont totalement dépourvus de fucose, il est possible d'utiliser des méthodes de préparation connues de l'homme du métier, comme par exemple celles décrites dans les documents EP1176195, US 7 214 775, US 6 994 292, US 7 425 449, US2010223686, WO2007099988, EP 1 705 251, cette liste n'étant pas limitative. Il peut s'agir par exemple d'une méthode utilisant une cellule hôte exprimant au moins un acide nucléique codant pour un fragment Fc, et dont la glycosylation est modifiée par délétion du gène codant pour l'$\alpha$1,6- fucosyltransferase ou par addition d'une mutation de ce gène pour éliminer l'activité $\alpha$1,6- fucosyltransferase, et exprimant à ce titre un fragment d'anticorps dépourvu de fucose.

### Applications thérapeutiques

En raison de leurs nombreux avantages, aussi bien en terme d'efficacité, que de fonctions effectrices optimisées ou d'effets secondaires réduits, les fragments Fc de la divulgation sont utiles pour leur utilisation dans le traitement d'une maladie auto-immune et/ou inflammatoire.

Selon la divulgation, des fragments Fc ayant une affinité altérée pour au moins un récepteur Fc, en une quantité thérapeutiquement efficace, peuvent être utilisés pour traiter une maladie auto-immune et/ou inflammatoire chez un patient.

Une maladie auto-immune et/ou inflammatoire selon la divulgation désigne une maladie auto-immune et/ou inflammatoire primitive ou secondaire, spécifique d'organe ou systémique, associée ou non à des auto-anticorps pathogènes.

Par exemple, la maladie selon la divulgation peut être choisie parmi le Purpura Thrombotique Thrombocytopénique (PTT), Purpura Thrombotique Idiopatique (PTI), le rejet de greffes ou d'organes, la maladie du greffon contre l'hôte, la polyarthrite rhumatoïde, le lupus érythémateux disséminé, les différents types de sclérose, le syndrome de Sjögren primitif (ou syndrome de Gougerot-Sjögren), les polyneuropathies auto-immunes comme la sclérose en plaques, le diabète de type I, les hépatites auto-immunes, la spondylarthrite ankylosante, le syndrome de Reiter, l'arthrite de goutte, la maladie coeliaque, la maladie de Crohn, la thyroïdite chronique d'Hashimoto (hypothyroïdie), la maladie d'Adisson, les hépatites auto-immunes, la maladie de Basedow (hyperthyroïdie), la colite ulcérative, les vascularites comme les vascularites systémiques associées aux ANCA (anticorps anti-cytoplasme des neutrophiles), les cytopénies auto-immunes et autres complications hématologiques de l'adulte et de l'enfant, telles que les thrombopénies auto-immunes aiguës ou chroniques, les anémies hémolytiques auto-immunes, la maladie hémolytique du nouveau-né (MHN), la maladie des agglutinines froides, le purpura thrombotique thrombocytopénique et l'hémophilie acquise auto-immune ; le syndrome de Goodpasture, les néphropathies extra-membraneuses, les affections cutanées bulleuses auto-immunes, la myasthénie réfractaire, les cryoglobulinémies mixtes, le psoriasis, l'arthrite chronique juvénile, les myosites inflammatoires, les dermatomyosites et les affections systémiques auto-immunes de l'enfant incluant le syndrome des antiphospholipides, la maladie des tissus conjonctifs, les différents types de sclérose, l'inflammation auto-immune pulmonaire, le syndrome Guillain-Barre, la maladie de Kawasaki, la MMN (Multifocal Motor Neuropathie), la polyradiculonévrite inflammatoire demyélisante chronique (PIDC), la thyroïdite auto-immune, le mellitis, le myasthenia gravis, la maladie autoimmune inflammatoire de l'oeil, la neuromyélite optique (maladie de Devic), les sclérodermies, le pemphigus, le diabète par insulinorésistance, la polymyosite, l'anémie de Biermer, la glomérulonéphrite, la maladie de Wegener, la maladie de Horton, la périarthrite noueuse et le syndrome de Churg et Strauss, la maladie de Still, la polychondrite atrophiante, la maladie de Behçet, la gammapathie monoclonale, la granulomatose de Wegener, le lupus, la rectocolite hémorragique, le rhumatisme psoriasique, la sarcoïdose, la colite collagène, la dermatite herpétiforme, la fièvre méditerranéenne familiale, la glomérulonéphrite à dépôts d'IgA, le syndrome myasthénique de Lambert-Eaton, l'ophtalmie sympathique, le syndrome de Fiessinger-Leroy-Reiter et le syndrome uvéo-méningo-encéphalique.

D'autres maladies inflammatoires sont également listées, comme par exemple le syndrome de détresse respiratoire aiguë (ARDS), l'arthrite septique aiguë, l'arthrite adjuvante, l'encéphalomyélite allergique, la rhinite allergique, la vasculite allergique, l'allergie, l'asthme, l'athérosclérose, l'inflammation chronique due à des infections bactériennes ou virales chroniques, la bronchopneumopathie chronique obstructive maladie chronique (MPOC), les maladies coronariennes, l'encéphalite, les maladies inflammatoires de l'intestin, l'ostéolyse inflammatoire, l'inflammation associée à des réactions d'hypersensibilité aiguë et retardée, l'inflammation associée à des tumeurs, une lésion nerveuse périphérique ou des maladies démyélinisantes, une inflammation associée à un traumatisme des tissus tels que les brûlures et l'ischémie, inflammation due à une méningite, le syndrome de défaillance multiviscérale (multiple organ dysfunction syndrome, MODS), la fibrose pulmonaire, septicémie et choc septique, syndrome de Stevens-Johnson, arthrite indifférenciée, et les spondylarthropathies indifférenciées.

Selon l'invention, la maladie auto-immune est le purpura thrombotique idiopathique (PTI).

Un des effets observés est notamment une limitation ou une diminution de la destruction des plaquettes observée dans la pathologie PTI et une limitation ou une diminution de la perte de la gaine de myéline des nerfs périphériques dans le PIDC.

Selon la divulgation, la maladie est une maladie inflammatoire, telle que notamment la maladie du greffon contre l'hôte. Dans ce cas, les fragments Fc selon la divulgation portent une mutation induisant une hypersialylation, telle qu'une délétion d'un acide aminé en position 294 (DEL294) ou 293 (DEL293).

Toute voie d'administration est envisagée, notamment des voies parentérales, telles que la voie intraveineuse, intramusculaire, sous-cutanée, intradermique, topique, ou par voie mucosale, par exemple par inhalation. Les voies entérales (orale, rectale) et intrathécale sont également possibles. De préférence, la voie intraveineuse est utilisée.

Les fragments Fc selon l'invention sont généralement formulés au sein de compositions pharmaceutiques comprenant des excipients pharmaceutiquement acceptables.

Les compositions pharmaceutiques peuvent se présenter sous toute forme galénique adaptée en fonction de la voie d'administration choisie.

Les compositions pharmaceutiques utiles selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc. Les compositions peuvent être formulées éventuellement au moyen de formes galéniques ou de dispositifs assurant une libération prolongée et/ou retardée. Pour ce type de formulation, on utilise avantageusement un agent tel que la cellulose, des carbonates ou des amidons.

Les doses administrées peuvent variées, entre autres, selon le poids et l'âge du patient, et la sévérité de la maladie, appréciée par l'homme du métier.

Dans un mode de réalisation préféré, le dosage des fragments Fc selon l'invention est dans la gamme d'environ 0.05mg/kg à environ 1g/kg de poids corporel, soit d'environ 20mg à environ 100g par jour pour un adulte. De préférence, le dosage est d'environ 330mg/kg à environ 660mg/kg par jour.

Les exemples suivants illustrent l'invention sans en limiter la portée.

### *Construction des vecteurs d'expression des Fc recombinants (WT et mutés) :*

La séquence du Fc recombinant (aa 221-447) a été clonée dans un vecteur d'expression eucaryote générique dérivé de pCEP4 (Invitrogen) pour l'expression dans les cellules HEK et le vecteur optiCHO pour l'expression dans les cellules YB2/0 en utilisant des protocoles de PCR standard.

Toutes les mutations d'intérêt dans le fragment Fc ont été insérées dans le vecteur d'expression par PCR de chevauchement en utilisant deux amorces contenant la mutation. Les fragments ainsi obtenus par PCR ont ensuite été associés et le fragment résultant a été amplifié par PCR en utilisant des protocoles standards. Le produit de PCR a été purifié sur gels d'agarose 1% (w/ v), digéré avec les enzymes de restriction adéquates et cloné dans le vecteur d'expression du Fc recombinant.

La Figure 7 montre la carte du vecteur pCEP4 utilisé pour l'expression du variant T5A-74 dans les cellules HEK, et la carte du vecteur OptiCHO utilisé pour l'expression du variant T5A-74 dans les cellules YB2/0.

*Production des Fc recombinants dans les cellules HEK*

Les cellules HEK 293 ont été transfectées avec le vecteur d'expression du Fc recombinant (WT ou mutés) pCEP4 selon des protocoles standards (Invitrogen). Les cellules ont été cultivées de manière à produire les anticorps de manière transitoire. Les anticorps produits ont pu être isolés et purifiés selon des techniques courantes de l'art, en vue de leur caractérisation. Les taux de production obtenus sont de l'ordre de 150 à 500 $\mu$g/ml. La pureté et qualité des produits a été vérifiée en SDS-PAGE et SEC.

*Production des Fc recombinants dans les cellules YB2/0*

Les cellules YB2/0 ont été transfectées de manière stable par électroporation avec le vecteur d'expression du Fc recombinant (WT ou mutés) optiCHO selon des protocoles standards. La production a été réalisée en pools stables ou après clonage de cellules YB2/0.

Les étapes de production par culture cellulaire et de purification des anticorps ont été réalisées selon des techniques courantes de l'art, en vue de leur caractérisation. Les taux de production obtenus sont de l'ordre de 3 à 30 $\mu$g/ml. La pureté et qualité des produits a été vérifiée en SDS-PAGE et SEC.

**Exemple 1 : Test d'efficacité (inhibition de la lyse de globules rouges) :**

Pour mimer la situation de lyse de globules rouges qu'on observe dans le purpura thrombopénique idiopathique (PTI), impliquant les autoanticorps du patient atteint du PTI, une lyse de globules rouges médiée par des cellules effectrices en présence d'un anticorps monoclonal anti-Rhesus D (RhD) a été conduite, et la capacité de différentes quantités d'immunoglobulines polyvalentes (IgIV) ou de fragments Fc recombinants, mutés (fragments Fc recombinants portant les mutations selon le **Tableau 1 ci-dessous)** et non mutés, à inhiber cette lyse, par exemple par compétition avec l'anti-RhD pour la fixation des récepteurs Fc à la surface des cellules effectrices, a été évaluée.

**Tableau 1 : Constructions de variants Fc mutés préférés de la divulgation**

Le tableau ci-dessous représente les différents variants de fragments Fc mutés qui ont été testés dans les expériences de test d'efficacité (inhibition de la lyse de globules rouges) et de test de liaison au FcRn et CD16aV humains par résonance plasmonique de surface (SPR).

| Nom | Variant | Mutation(s) ajoutée(s) |
|---|---|---|
| T5A-74I | T5A-74 | T260A |
| T5A-74J | T5A-74 | E258I |
| T5A-74K | T5A-74 | K290Y |
| T5A-74L | T5A-74 | E294A |
| T5A-74M | T5A-74 | Y296W |
| ZAC2-85 | WT | T260A |
| ZAC3-172 | WT | K290Y |
| A3A-184A | WT | K334N, P352S, V397M, A378V |
| A3A-105D | WT | G316D, K326E, A378V |
| J3B-118A | WT | P396L, N421T, A378V |

Pour cela, à titre de cellules effectrices, des PBMC (cellules mononucléaires du sang périphérique) ont été purifiées à partir du sang périphérique par gradient de Ficoll. Les globules rouges Rh-D+ obtenus à partir de donneurs de sang sains Rh-D+ ont été mélangés avec un anticorps monoclonal anti-RhD. Les PBMC ont été incubées avec des globules rouges Rh-D+ opsonisés (rapport effecteur / cible de 2/1).

Pour évaluer la capacité des candidats (IgIV et fragments Fc recombinants, mutés définis selon le Tableau 1 et non muté) à inhiber une cytotoxicité induite par un anticorps anti-RhD par compétition et/ou saturation des récepteurs Fc, diverses concentrations des candidats (0 à 9,75 $\mu$M) ont été ajoutées à chaque puits. Après 16 heures, le pourcentage de globules rouges lysés a été estimé de façon chromogénique en mesurant la quantité d'hémoglobine libérée dans les surnageants (mesure de densité optique ou DO). La lyse spécifique est calculée en pourcentage selon la formule

suivante :

$$\text{DO échantillon} - \text{DO témoin 0\%} / \text{DO témoin 100\%} - \text{DO témoin 0\%} * 100 = \% \text{ Lyse}$$

dans laquelle :

« DO témoin 100% » correspond à la lyse totale des hématies (par exemple $NH_4Cl$)

« DO témoin 0% » correspond à la lyse observée avec un mélange réactionnel sans anticorps Les résultats sont exprimés en % de lyse spécifique (cf Figures 4 et 5).

**Exemple** 2 : **Test de liaison au FcRn et CD16aV humains par résonance plasmonique de surface (SPR) sur Biacore X100** :

Protéines utilisées :

Le FcRn humain recombinant (FcRn a et b2 microglobuline) a été produit en cellules baculovirus par la société GTP Technology (Labège, France) comme décrit précédemment (Popov et al., Mol. Immunol. 33:521-530 (1996)). Le CD16aV humain recombinant est disponible commercialement (R&D system).

Test sur FcRn :

Les cellules FC1 et FC2 d'une puce CM5 (Biacore, GE Healthcare) ont été activées pendant 3 minutes avec un mélange 1/1 de N-hydroxysuccinimide 0,1 M et de 3-(N,N-dimethylamino)propyl-N-ethylcarbodiimide 0,1 M à 30 $\mu$l/min. Le FcRn recombinant a ensuite été immobilisé sur la cellule FC2 à 32,6 $\mu$g/mL en tampon Acétate de Na 10 mM pH 5 (immobilisation pendant 100 secondes, taux d'immobilisation final de 350 RU). La cellule FC1 a été utilisée comme contrôle négatif, préparée de la même manière que la FC2 mais en absence de FcRn recombinant. Les Fc recombinants à tester ont été injectés à 6 concentrations différentes (300nM, 150nM, 75nM, 30nM, 15nM et 0) en tampon phosphate Na 50mM, NaCl 150 mM, tween20 0,05% pH6 pendant 8 minutes à 10 $\mu$l/min sur les cellules FC1 et FC2. Les cellules FC1 et FC2 ont été régénérées entre chaque concentration d'échantillon par une injection pendant une minute de tampon phosphate Na 50mM, NaCl 150 mM, tween20 0,05% pH7,8. Les données générées ont été analysées avec le logiciel BIAevaluation version 3.1 (Biacore), en soustrayant le signal contrôle obtenu sur FC1 du signal test obtenu sur FC2.

Test sur CD16aV :

Les cellules FC1 et FC2 d'une puce CM5 (Biacore, GE Healthcare) ont été préparées avec le kit His Capture de GE Healthcare référence 28-9950-56, de manière à immobiliser l'anticorps anti-Histidine à 50 $\mu$g/ml (débit de 5 $\mu$l/min, activation en EDC/NHS pendant 7 min, immobilisation pendant 7 minutes, taux d'immobilisation finale de 12000 RU sur FC1 et FC2). Le CD16aV recombinant humain a été immobilisé sur la cellule FC2 (1 $\mu$g/ml en HBS-EP+, Biacore, GE Healthcare) pendant 60 secondes à 5 $\mu$l/min. Les Fc recombinants à tester ont été injectés sur les cellules FC1 et FC2 à 5 concentrations différentes (1000nM, 500nM, 250nM, 125nM et 25nM) en tampon HBS-EP+ en condition SCK (Single Cycle Kinetics), avec un contact de 60 secondes, une dissociation de 300 secondes et un débit de 30 $\mu$l/min sans régénération entre chaque concentration. La régénération finale, entre chaque Fc recombinant, a été réalisée en tampon Glycine 10mM pH 1,5 pendant 60 secondes à 30 $\mu$l/min sur les cellules FC1 et FC2. Les données générées ont été analysées avec le logiciel BIAevaluation version 3.1 (Biacore), en soustrayant le signal contrôle obtenu sur FC1 du signal test obtenu sur FC2.

**Revendications**

1. Composition comprenant des fragments Fc d'anticorps, pour une utilisation dans le traitement du purpura thrombotique idiopathique, lesdits fragments Fc étant des fragments Fc isolés ayant une affinité améliorée d'un ratio au moins égal à 2 pour le récepteur FcRγIII (CD16a), par rapport à un fragment Fc parent d'IgG1 sauvage de séquence SEQ ID NO :1, et comprenant au moins la séquence SEQ ID NO :19.

2. Composition pour son utilisation selon la revendication 1, dans laquelle les fragments Fc consistent en la séquence SEQ ID NO :19.

3. Composition comprenant des fragments Fc pour une utilisation selon l'une des revendications 1 ou 2, dans laquelle les fragments Fc sont obtenus par voie recombinante.

4. Composition comprenant des fragments Fc pour une utilisation selon l'une des revendications 1 à 3, lesdits fragments Fc possédant sur leur site de glycosylation (Asn 297) des N-glycannes, **caractérisée en ce que** lesdits N-glycannes des fragments Fc présentent un taux de fucosylation inférieur à 65%, de préférence inférieur à 60%, de préférence inférieur à 55%, de préférence inférieur à 50%, de préférence encore inférieur à 45%, de préférence inférieur à 40%, de préférence inférieur à 35%, de préférence inférieur à 30%, de préférence inférieur à 25%, de préférence inférieur à 20%.

5. Composition comprenant des fragments Fc pour une utilisation selon l'une des revendications 1 à 4, lesdits fragments Fc possédant sur leur site de glycosylation (Asn 297) des N-glycannes, **caractérisée en ce que** lesdits N-glycannes des fragments Fc présentent une structure glycannique de type biantenné, avec des chaînes courtes, une faible sialylation, présentant des mannoses terminaux et/ou des N-acétylglucosamines terminaux non intercalaires.

6. Composition comprenant des fragments Fc pour une utilisation selon l'une des revendications 1 à 4, lesdits fragments Fc possédant sur leur site de glycosylation (Asn 297) des N-glycannes, **caractérisée en ce que** lesdits N-glycannes des fragments Fc présentent une teneur supérieure à 60 % pour les formes G0+G1+G0F+G1F, les formes G0F+G1F étant inférieures à 50 %.

7. Composition comprenant des fragments Fc pour une utilisation selon l'une des revendications 1 à 5, lesdits fragments Fc possédant sur leur site de glycosylation (Asn 297) des N-glycannes, **caractérisée en ce que** lesdits N-glycannes des fragments Fc présentent une teneur supérieure à 60 % pour les formes G0+G1+G0F+G1F, la teneur en fucose étant inférieure à 65 %.

8. Composition comprenant des fragments Fc pour une utilisation selon la revendication 6 ou 7, lesdits fragments Fc possédant sur leur site de glycosylation (Asn 297) des N-glycannes, **caractérisée en ce que** lesdits N-glycannes des fragments Fc présentent une teneur inférieure à 40% pour les formes G1F+G0F.

**Patentansprüche**

1. Zusammensetzung, umfassend Fc-Fragmente von Antikörpern für eine Verwendung bei der Behandlung des immune Thrombozytopenie, wobei die Fc-Fragmente isolierte Fc-Fragmente mit einer verbesserten Affinität eines Verhältnisses von mindestens gleich 2 für den Empfänger FcRyIII (CD16a) mit Bezug auf ein Eltern-Fc-Fragment von Wildtyp IgGI mit der Sequenz SEQ ID NO : 1 sind, und umfassend mindestens die Sequenz SEQ ID NO :19.

2. Zusammensetzung für ihre Verwendung nach Anspruch 1, wobei die Fc-Fragmente aus der Sequenz SEQ ID NO :19 bestehen.

3. Zusammensetzung, umfassend Fc-Fragmente für eine Verwendung nach einem der Ansprüche 1 oder 2, wobei die Fc-Fragmente auf rekombinantem Weg erhalten werden.

4. Zusammensetzung, umfassend Fc-Fragmente für eine Verwendung nach einem der Ansprüche 1 bis 3, wobei die Fc-Fragmente an ihrer Glykolysierungsstelle (Asn 297) N-Glykane aufweisen, **dadurch gekennzeichnet, dass** die N-Glykane der Fc-Fragmente einen Fukosylierungsgrad von weniger als 65 %, vorzugsweise weniger als 60 %, vorzugsweise weniger als 55 %, vorzugsweise weniger als 50 %, noch bevorzugter weniger als 45 %, vorzugsweise weniger als 40 %, vorzugsweise weniger als 35 %, vorzugsweise weniger als 30 %, vorzugsweise weniger als 25 %, vorzugsweise weniger als 20 % aufweisen.

5. Zusammensetzung, umfassend Fc-Fragmente für eine Verwendung nach einem der Ansprüche 1 bis 4, wobei die Fc-Fragmente an ihrer Glykosylierungsstelle (Asn 297) N-Glycane aufweisen, **dadurch gekennzeichnet, dass** die N-Glykane der Fc-Fragmente eine Glykanstruktur vom biantenären Typ aufweisen, mit kurzen Ketten, einer schwachen Sialylierung, mit terminalen Mannosen und/oder nicht zwischengeschaltete terminale N-acetylglukosamine aufweist.

**6.** Zusammensetzung, umfassend Fc-Fragmente für eine Verwendung nach einem der Ansprüche 1 bis 4, wobei die Fc-Fragmente auf ihrer Glykosylierungsstelle (Asn 297) N-Glycane aufweisen, **dadurch gekennzeichnet, dass** die N-Glykane der Fc-Fragmente einen Gehalt von mehr als 60 % bei den Formen G0+G1+G0F+G1F aufweisen, wobei die Formen G0F+G1F weniger als 50 % sind.

**7.** Zusammensetzung, umfassend Fc-Fragmente für eine Verwendung nach einem der Ansprüche 1 bis 5, wobei die Fc-Fragmente an ihrer Glykosylierungsstelle (Asn 297) N-Glycane aufweisen, **dadurch gekennzeichnet, dass** die N-Glykane der Fc-Fragmente einen Gehalt von mehr als 60 % bei den Formen G0+G1+G0F+G1F aufweisen, wobei der Fukosegehalt weniger als 65 % ist.

**8.** Zusammensetzung, umfassend Fc-Fragmente für eine Verwendung nach einem der Ansprüche 6 oder 7, wobei die Fc-Fragmente an ihrem Glykosylierungsstelle (Asn 297) N-Glycane aufweisen, **dadurch gekennzeichnet, dass** die N-Glykane der Fc-Fragmente einen Gehalt von weniger als 40 % bei den Formen G1F+G0F aufweisen.

**Claims**

**1.** Composition comprising Fc antibody fragments for use in the treatment of immune thrombocytopenic purpura, said Fc fragments being isolated Fc fragments having improved affinity, by a ratio of at least 2, for the FcR$\gamma$III(CD16a) receptor compared with a parent Fc fragment of wild-type IgGI of sequence SEQ ID NO: 1, and comprising at least the sequence SEQ ID NO: 19.

**2.** Composition for use according to claim 1, wherein the Fc fragments are composed of the sequence SEQ ID NO:19.

**3.** Composition comprising Fc fragments for use according to one of claims 1 or 2, wherein the Fc fragments are obtained via recombinant route.

**4.** Composition comprising Fc fragments for use according to one of claims 1 to 3, said Fc fragments having N-glycans on their glycosylation site (Asn 297), **characterized in that** said N-glycans of the Fc fragments have a fucosylation rate lower than 65 %, preferably lower than 60 %, preferably lower than 55 %, preferably lower than 50 %, more preferably lower than 45 %, preferably lower than 40 %, preferably lower than 35 % , preferably lower than 30 %, preferably lower than 25 %, preferably lower than 20 %.

**5.** Composition comprising Fc fragments for use according to one of claims 1 to 4, said Fc fragments having N-glycans on their glycosylation site (Asn 297), **characterized in that** said N-glycans of the Fc fragments have a glycan structure of biantennary type with short chains, weak sialylation, having non-intercalating terminal mannoses and/or terminal N-acetylglucosamines.

**6.** Composition comprising Fc fragments for use according to one of claims 1 to 4, said Fc fragments having N-glycans on their glycosylation site (Asn 297), **characterized in that** the content of said N-glycans in the Fc fragments is higher than 60 % for the forms G0+G1+G0D+G1D, and lower than 50 % for the forms G0F+G1F.

**7.** Composition comprising Fc fragments for use according to one of claims 1 to 5, said Fc fragments having N-glycans on their glycosylation site (Asn 297), **characterized in that** the content of said N-glycans in the Fc fragments is higher than 60 % for the forms G0+G1+G0F+G1F, the fucose content being lower than 65 %.

**8.** Composition comprising Fc fragments for use according to claim 6 or 7, said Fc fragments having N-glycans on their glycosylation site (Asn 297), **characterized in that** the content of said N-glycans in the Fc fragments is lower than 40 % for the forms G1 F+G0F.

Figure 1

A

NH2

COOH

COOH

NH2

B

NH2

COOH

## Figure 2

**G0,**

**G0F,**

**G1, et**

**G1F**

GlcNAc        Mannose        Fucose        Galactose

## Figure 3

Figure 4

2126 16 201-258

Figure 5

Figure 6

CMVie enhancer

beta Actin core promoter

Poly A TK

R-U5'

neo

elF4G1

elF4g1-1 (100.0%)

pTK

*Nhd* (1343)

MB7

pUC origin

hinge partiel

CH2

**FcT5A74-HEK**

CH3

**11487 bp**

*Swd* (2099)

*Xho*I (2104)

SV40-3'UT R (100.0

AmpR

SV40 pA signal

OriP

EBNA-1    1

2

*Bg*/II (1)

Amp r

CMVie enhancer

CDK9 core promoter

elF4G1muté

*Bg*/II (1360)

intron EFss

Col E1 ori

**FcT5A74-CHO**

5'ef1a (100.0%

**6942 bp**

*Nhd* (1963)

MB7

hinge partiel

polyA synthétique

CH2

CH3

néo

*Swd* (2719)

SV40 Enh-prom préc

*Xbd* (2730)

2BGHPA (100.0%)

BGH polyA

# Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004099374 A
- EP 2233500 A
- WO 2010106180 A
- WO 0177181 A
- WO 02038756 A
- WO 200748077 A
- US 7700321 B
- US 2010291628 A
- US 20090228994 A
- EP 1500698 A
- EP 1792987 A

- US 7846725 B
- US 7393683 B
- WO 2006133148 A
- WO 0200879 A
- EP 1176195 A
- US 7214775 B
- US 6994292 B
- US 7425449 B
- US 2010223686 A
- WO 2007099988 A
- EP 1705251 A

**Littérature non-brevet citée dans la description**

- **KABAT et al.** Séquences de protéines d'intérêt immunologique. Public Health Service, National Institutes of Health, 1991
- Basic and Clinical Immunology. Appleton & Lange, 1994, 71
- Basic and Clinical Immunology
- **RAVETCH** ; **KINET**. *AnnualReview of Immunology*, 1991, vol. 9, 457-492
- **UANANUE** ; **BENACERRAF**. Textbook of Immunology. Williams & Wilkins, 1984, 218
- **MUTA T et al.** *Nature*, 1994, vol. 368, 70-73

- **AYALA M** ; **GAVILONDO J** ; **RODRIGUEZ M** ; **FUENTES A** ; **ENRÍQUEZ G** ; **PÉREZ L** ; **CREMATA J** ; **PUJOL M**. Production of plantibodies in Nicotiana plants. *Methods Mol Biol*, 2009, vol. 483, 103-34
- **POLLOCK, D.P** ; **J.P. KUTZKO** ; **E. BIRCK-WILSON** ; **J.L. WILLIAMS** ; **Y. ECHELARD** ; **H.M. MEADE**. Transgenic milk as a method for the production of recombinant antibodies. *Journal of Immunological Methods.*, 1999, vol. 231, 147-157
- **POPOV et al.** *Mol. Immunol.*, 1996, vol. 33, 521-530